Europäisches Patentamt

European Patent Office

Office européen des brevets

(19)

(11) Publication number: 0 299 078

A1

(12) EUROPEAN PATENT APPLICATION
published in accordance with Art. 158(3) EPC

(21) Application number: 88901097.1

(22) Date of filing: 22.01.88

Data of the international application taken as a basis:

(86) International application number:
PCT/JP88/00044

(87) International publication number:
WO88/05267 (28.07.88 88/17)

(51) Int. Cl.³: A 23 L 1/28
A 23 L 1/229

(30) Priority: 22.01.87 JP 11226/87

(43) Date of publication of application:
18.01.89 Bulletin 89/3

(84) Designated Contracting States:
CH DE FR GB IT LI NL

(71) Applicant: Kohjin Co., Ltd.
No. 1-1, Shinbashi 1 chome
Minato-ku Tokyo-to(JP)

(72) Inventor: HARADA, Shigeyoshi
11772-81, Aza Nooka
Saiki-City Ooita 876(JP)

(72) Inventor: ITOU, Juniti
802, Ikeda
Saiki-City Ooita 876(JP)

(72) Inventor: YANO, Masao
3-17, Harayama 4-chome, Urawa-shi
Saitama-ken(JP)

(72) Inventor: AOYAGI, Yoshiki
11772-81, Aza Nooka, Saiki-shi
Ooita-ken(JP)

(72) Inventor: MAEKAWA, Hirokazu
27-16, Higashi-machi, Saiki-shi
Ooita-ken(JP)

(74) Representative: Thomsen, Dieter, Dr.rer.nat.
Kaiser-Ludwig-Platz 6
D-8000 München 2(DE)

(54) YEAST EXTRACT AND PROCESS FOR ITS PREPARATION.

(57) The process comprises extracting an essential component and RNA-containing yeast cells, decomposing the RNA into nucleotides using 5'-phosphodiesterase and, if desired, acting deaminase to prepare yeast extract. In this process, a cell strain capable of yielding crude RNA in a high content is used and, upon decomposition of RNA into nucleotides, and aqueous solution containing the yeast cells is previously heat-treated at 80 to 120°C to thereby convert the whole of the RNA to tasty 5'-nucleotide while inhibiting its conversion into 2'- or 3'-nucleotide. In this conversion, an alkali, common salt, and a surfactant are not necessarily used in large amounts unlike in the prior art, and hence the whole extract component can be directly converted into a seasoning material containing 5'-GMP and 5'-IMP in large amounts. Particularly, a yeast extract obtained by using 10 % or more, preferably 15 % or more, yeast variant provides a seasoning material with a particularly excellent taste. A novel yeast strain containing RNA in a large amount, *Candida utilis* CSB 6316, is also disclosed.

Croydon Printing Company Ltd.

## YEAST EXTRACT AND PROCESS FOR PREPARING THE SAME

Technical Field:

This invention relates to a process for preparing a yeast extract containing natural inosine 5'-monophosphate (5'-IMP), guanosine 5'-monophosphate (5'-GMP) or the like at a high concentration from an RNA-containing extract which is obtained from RNA-rich living yeast cells, the yeast extract thus obtained, and an RNA-rich mutant yeast strain available in said process.

Background Art:

There are various yeast extracts which are commercially available as seasonings. These yeast extracts are generally prepared from Baker's yeast or Brewer's yeast. These extracts may be obtained by directly extracting yeast cells as such with hot water, decomposing the same with an enzyme, mechanically grinding the same, or autolyzing the same. The products are in the form of a powder or a paste. Other natural seasonings include extracts of meat fish, vegetables or seaweeds. On the other hand, a nucleic acid seasoning, which is obtained by extracting and purifying RNA from an RNA-rich torula yeast, then chemically or enzymatically decomposing the RNA, and fractionating and purifying 5'-IMP and 5'-GMP from a mixture of 5'-nucleotides thus obtained, has been widely applied.

However each of the commercially available yeast extracts as described above is unsatisfactory in the flavor, since it contains a large amount of adenine 3'-monophosphate (3'-AMP) and guanosine 3'-monophosphate (3'-GMP) but scarcely any 5'-IMP and 5'-GMP which contribute to an excellent flavor.

There has been established a process which comprises efficiently extracting RNA from yeast cells by using large amounts of alkalis, common salt or surfactants and decomposing the obtained RNA into 5'-IMP and 5'-GMP. In this case, these additives are washed away after extracting the RNA. Thus the decomposition products, i.e., nucleotides contain no yeast extract components.

As described above, each of the conventional yeast extracts has only an unsatisfactory flavor. Thus it is generally necessary to add artificial seasoning compounds such as 5'-IMP or 5'-GMP, which are separately prepared, to the yeast extract in order to enhance its taste, thereby becoming itself an artificial seasoning.

DETAILED DESCRIPTION OF THE INVENTION

Means for Solving the Problems:

The present inventors have attempted to overcome these problems and have completed the present invention. Accordingly, the present invention relates to a process for preparing a natural yeast extract rich in 5'-GMP optionally together with 5'-IMP, which comprises heating an aqueous solution of living yeast cells containing a large amount of RNA at 80 to 120°C and treating the heated solution with 5'-phosphodiesterase and then optionally with deaminase, the yeast extract thus obtained, and an RNA-rich mutant yeast strain.

The yeast extract according to the present invention exhibits a unique flavor, i.e., highly spreadable, full bodied and yet scarcely yeasty odor.

Now the present invention will be described in detail.

The origins of the 5'-phosphodiesterase, deaminase and protease to be used in the present invention are not particularly restricted. Thus commercially available ones may be employed therefor.

First, a yeast strain is inoculated into a known medium and cultured therein. The obtained cells are thoroughly washed and suspended in water 5 to 30 times

- 4 -

by weight as much as the dry cells.  The resulting suspension is treated at 80 to 120°C, preferably 90 to 100°C, to inactivate ribonucleases in the cells.

Living yeast cells are to be used in the present invention since dry yeast cells, which have been subjected to a severe thermal treatment, might give a final product of an undesirable flavor.

The yeast cell  preferably contains 10 % by weight or above  on a dry basis , still preferably 15 % by weight or above, of RNA.

Examples of the RNA-rich yeast strains include Candida utilis CS 7529 (FERM BP-1656) and Candida utilis CSB 6316 (FERM BP-1657).

The above mutant strain CSB 6316 is obtained by treating a cold-sensitive parent strain CS 7529 with nitrosoguanidine which is a mutagen.  The aimed strain can be obtained by selecting a mutant strain which can grow in a synthetic medium containing boric acid at such a high concentration that the parent strain CS 7529 can not grow therein, and it contains RNA at a high concentration.

The mycological properties of Candida utilis CS 7529 and Candida utilis CSB 6316 are as follows.  These two strains have the same mycological properties except the resistance to boric acid. Further the mycological properties of these strains are the same as those of <u>Candida</u> <u>utilis</u> except the resistance to boric acid and cold-sensitivity.

Mycological properties of <u>Candida</u> <u>utilis</u> CS 7529 and CSB 6316:

1. Each strain forms fully edged, convex, smooth, dimly gloss and soft colonies of a creamy color on a YM agar medium.

It shows clouding and precipitation in a liquid medium containing glucose, yeast extract and peptone, though neither pellicle nor fairy ring is observed.

2. It forms pseudohyphae on a Darmot plate.

3. It forms no ascospores in sodium acetate slant agar, malt extract slant agar, V8 juice slant agar and corn milk slant agar media.

4. It ferments glucose, sucrose and raffinose but not galactose, maltose, trehalose nor lactose.

5. It assimilates glucose, sucrose, maltose, cellobiose, trehalose, raffinose, melezitose, xylose, ethanol, mannitol, lactic acid, citric acid and $KNO_3$.

6. It grows well in a medium free from vitamins.

7. It shows ellipsoidal or annular cells (3.5 to 4.5 x 7 to 13 µm), when cultured at 30°C for three days.

8. It shows a little growth but scarcely any multiplication at 20°C.

9. It grows well at 30 to 40°C.

The resistances to boric acid of the CS 7529 and CSB 6316 strains are as follows.

Each strain is cultured batchwise in a medium containing 3 % by weight of glucose, 0.3 % by weight of monobasic potassium phosphate, 0.7 % by weight of ammonium sulfate, 0.03 % by weight of magnesium sulfate, 5 ppm of ferric sulfate, 9 ppm of zinc sulfate, 4 ppm of manganese chloride, 100 to 1000 ppm of $B^{3+}$ and 2 % of agar on a plate at 30°C for seven days. Table 1 shows the results of resistance to boric acid of each strain.

Table 1

| $B^{3+}$ conc. (ppm) | 100 | 500 | 1000 |
|---|---|---|---|
| CS 7529 | + | - | - |
| CSB6316 | + | + | + |

A strain containing a small amount of RNA can give a yeast extract containing only a limited amount of flavoring components such as 5'-IMP and 5'-GMP. A seasoning obtained in the above-mentioned yeast extract has a yeasty odor and less spreadable flavor due to a low content balance

- 7 -

against other extracted components.

When the thermal treatment as described above is carried out at a temperature lower than 80°C, the ribonucleases can not be sufficiently inactivated. In this case, RNA would be converted into 2'- or 3'-nucleotides or nucleosides, which would inhibit the formation of the preferable flavor components, i.e., 5'-nucleotides. On the other hand, a thermal treatment at a temperature exceeding 120°C would cause an undesirable scorching odor.

After inactivating the ribonucleases in the cells, the cell suspension is treated with protease, if required. Alternately an alkali is added thereto. Then the RNA components are extracted. This extract is treated with 5'-phosphodiesterase and then optionally with deaminase.

The protease is added in an amount of 1 g/$\ell$ or less and allowed to act at 30 to 50°C for 1 to 20 hours. Even if the amount thereof exceeds 1 g/$\ell$, the effect could be hardly elevated. On the other hand, when a smaller amount of the enzyme is added, the effect would be lowered, which causes a decrease in the amount of the RNA decomposition products, i.e., 5'-nucleotides at the subsequent step.

Examples of the alkali include caustic soda and caustic potash. This alkali treatment may be carried out at a pH value of 8 to 12, preferably 9 to 10, and at a temperature of 40 to 50°C for one to three hours. When the pH value is lower than 8, the RNA can not be sufficiently extracted and the employed alkali can hardly exert the aimed effect. On the other hand, when it exceeds 12, an undesirable alkali decomposition of the RNA might occur. It is preferable to add the alkali in an amount as small as possible to thereby minimize the effect of the salt on the taste of the final product.

Then 5'-phosphodiesterase is added to the extract to thereby decompose the RNA contained therein into 5'-nucleotides. This enzyme is usually added in an amount of 0.1 to 0.5 g/ℓ and allowed to act at 50 to 80°C for 30 minutes to three hours. It is preferable to heat the extract to 50 to 80°C, still preferably 60 to 70°C, prior to the addition of the 5'-phosphodiesterase. When the enzyme is added at a temperature lower than 50°C a small amount of phosphatase, which is frequently contained in a 5'-phosphodiesterase preparation, might cause the formation of impure by-products. 5'-AMP present in the obtained solution containing the 5'-nucleotides may be converted into 5'-IMP, if desired, by adding 0.1 to 0.5 g/ℓ of

deaminase thereto and allowing the enzyme to act at 35 to 45°C for 30 minutes to two hours.

After the above-mentioned procedure, the mixture is heated to 90 to 100°C for 30 to 60 minutes to inactivate the employed enzymes. Then the solid matters are removed by, for example, centrifugation and the supernatant is concentrated to give a paste or further dried to give a powder. Although the concentration or drying method is not particularly restricted, those effected at a moderate temperature, such as concentration under reduced pressure or spray drying, are preferable.

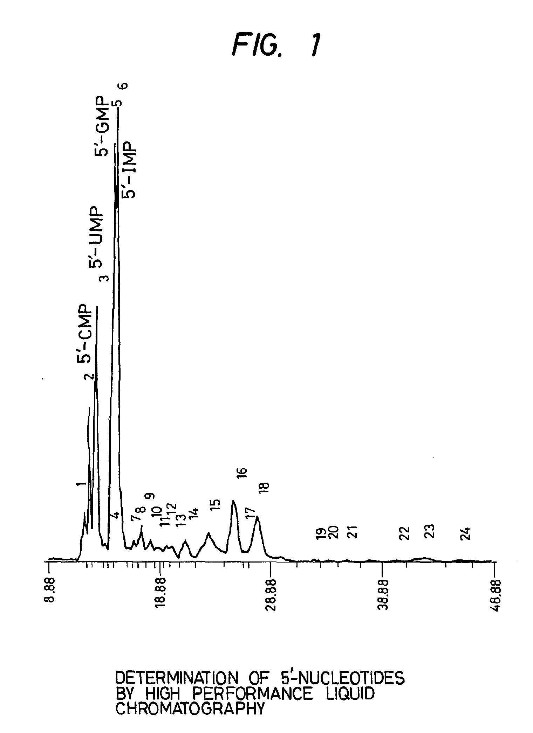

Fig. 1 is a chart showing the result of the determination of the yeast extract as prepared in Example 3 by high performance liquid chromatography. Best Mode of Carrying out the Invention:

To further illustrate the present invention, but not meaning limitation thereon in any way, the following Examples will be given.

Example 1

Candida utilis CSB 6316 was preliminarily cultured in a flask to give a seed culture, which was then inoculated into a 30-ℓ fermenter at a concentration of 2 %.

It was cultured batchwise in a medium containing 3 % by weight of glucose, 0.3 % by weight of monobasic potassium phosphate, 0.7 % by weight of ammonium sulfate, 0.03 % by weight of magnesium sulfate, 5 ppm of ferric sulfate, 9 ppm of zinc sulfate and 4 ppm of manganese chloride for 16 hours.

The culture was carried out under the following conditions: liquid content of the fermentor: 15 $\ell$, temperature: 30°C, aeration rate: 15 $\ell$/min, agitator speed: 500 rpm and pH value: 4.0 (automatically controlled with ammonia). The parent strain of the above variant was cultured under the same conditions as those defined above. Table 2 shows the results of the examination of the cells thus obtained.

Table 2

| Strain | Culture time (hr) | Cell conc. (%) | RNA content (%) |
|--------|-------------------|----------------|-----------------|
| CSB 6316 | 14 | 0.29 | 17.1 |
|          | 16 | 0.64 | 17.0 |
| CS 7529 | 14 | 0.30 | 14.3 |
|         | 16 | 0.65 | 14.1 |

Example 2

A boric acid resistant variant _Candida_ _utilis_ CSB 6316 and the parent strain thereof were cultured batchwise each in a 30-$\ell$ fermentor under the same conditions as those defined in Example 1. In the longarithmic growth phase, each culture was converted into a continuous one. The same medium as the one described in Example 1 was continuously added to the culture under the following conditions: pH value: 4.0, temperature: 30°C, liquid content of the fermentor: 15 $\ell$, agitator speed: 500 rpm and aeration rate: 15 $\ell$/min.

The parent strain of the above variant was cultured in a conitnuous manner under the same conditions as those defined above. Table 3 shows the results.

Table 3

| Strain | Specific growth rate ( /hr) | Cell conc. (%) | RNA content (%) |
|--------|------------------------------|----------------|-----------------|
| CSB 6316 | 0.3 | 1.31 | 14.9 |
|  | 0.4 | 1.45 | 17.0 |
|  | 0.5 | 1.24 | 17.4 |
| CS 7529 | 0.3 | 1.32 | 12.4 |
|  | 0.4 | 1.44 | 14.3 |
|  | 0.5 | 1.25 | 14.8 |

The results of Examples 1 and 2 suggest that a remarkable increase in the RNA content was observed in the boric acid-resistant strain CSB 6316.

Example 3

1 ℓ of a prepropagation liquor of <u>Candida</u> <u>utilis</u> CS 7529 was inoculated into 15 ℓ of a medium, which was obtained by adjusting a pH value of an aqueous solution containing 5 % by weight of glucose, 0.2 % by weight of ammonium phosphate, 0.2 % by weight of ammonium sulfate, 0.1 % by weight of magnesium sulfate, 0.17 % by weight of potassium chloride, 5 ppm of ferric sulfate, 9 ppm of zinc sulfate and 4 ppm of manganese chloride to 4.5, in a 30-ℓ jar fermenter. Then the strain was cultured therein at 30°C for 24 hours to thereby give yeast cells.

After the completion of the culture, the cells were collected with a Sharples-type centrifuge to thereby give wet yeast cells, which were then suspended in water again and centrifuged. This procedure was repeated twice. Thus approximately 300 g, on a dry basis, of the cells were obtained. Water was added to these cells to give a total volume of 1500 mℓ. The obtained mixture was heated on a water bath. When the liquid temperature exceeded 80°C, heating was continued at 80 to 100°C for 30 minutes. Then the mixture was immediately cooled with running water until the liquid temperature reached 40°C. Subsequently 1.5 g of Protin PC-10 (a protease preparation mfd.

by Daiwa Kasei K.K.) dissolved in a small amount of water was added thereto and the resulting mixture was allowed to react under stirring at 30 to 50°C for ten hours.

Then the mixture was heated at a liquid temperature of 80°C or above for 30 minutes and subsequently cooled to 65°C. 0.2 g of Ribonuclease P (a 5'-phosphodiesterase preparation mfd. by Amano Pharmaceutical Co., Ltd.) dissolved in a small amount of water was added thereto and the resulting mixture was slowly stirred at this temperature for three hours. Then the mixture was cooled to 45°C and 0.2 g of Deamizyme (a deaminase preparation mfd. by Amano Pharmaceutical Co., Ltd.) was added thereto in the same manner as the one described above. The resulting mixture was maintained at this temperature under stirring for two hours. The insoluble matters were removed by centrifugation to thereby give an extract. This extract was heated to 90 to 95°C for 30 minutes, allowed to cool and powdered by spray drying. Thus approximately 75 g of a powdery yeast extract containing 12 % by weight of 5'-IMP, 8.3 % by weight of 5'-GMP, 6 % by weight of 5'-CMP and 11.8 % by weight of 5'-UMP was obtained. Each 5'-nucleotide was determined by high performance liquid chromatography under the following conditions:

packing: TSK-GEL ODS-80TM,

column: 15 cm x 4.6 mm (diameter),

eluent: 0.05 M $KH_2PO_4$ containing 7.0 by weight of methanol,

elution speed: 0.5 m$\ell$/min,

injected sample: 20 $\mu\ell$, and

detection: $UV_{254}$

The above conditions were also employed in the following Examples. A 1 % aqueous solution of this extract was not yeasty odor but showed a unique, complicated and desirable flavor wherein the amino acid and peptide-like taste originating from the yeast was blended with deliciousness similar to those of dried bonito and shiitake mushroom.

Example 4

The procedure of Example 3 was followed to give approximately 300 g of cells on a dry basis. Water was added to these cells to give a total volume of 1500 m$\ell$ and the resulting mixture was heated in the same manner as the one described in Example 3 and then cooled to a liquid temperature of 50°C. Subsequently 6 N caustic soda was added dropwise thereto under stirring to thereby give a pH value of 10. After stirring for additional three hours, the mixture was centrifuged to thereby remove the cells. The pH value of the supernatant thus obtained was immediately adjusted to 6 by adding 6 N hydrochloric acid thereto. The supernatant was heated to 65°C. Then it was treated with 5'-phosphdiesterase and deaminase in the same manner as the one described in Example 3 and then powdered by spray drying. Thus approximately 70 g of a powdery yeast extract containing 10.2 % by weight of 5'-IMP, 7.1 % by weight of 5'-GMP, 5.1 % by weight of 5'-CMP and 10.0 % by weight of 5'-UMP was obtained.

A 1 % aqueous solution thereof showed a flavor which was somewhat salty but almost the same as that of Example 3.

Example 5

Candida utilis CSB 6316 was cultured under aeration in the same medium as the one described in Example 3 at 30°C for 24 hours to thereby give yeast cells.

After the completion of the culture, the cells were treated in the same manner as the one described in Example 3 to thereby give wet cells. These cells were suspended in water again and centrifuged. This procedure was repeated twice. Thus approximately 360 g of the cells were obtained. The cells were successively treated with Protein PC-10, Ribonuclease P and Deamizyme, similar to the procedure of Example 3.

Then the reaction mixture was centrifuged to thereby remove the insoluble matters. The extract thus obtained was heated to 90 to 95°C for 30 minutes, allowed to cool and then powderd by spray drying. Thus approximately 98 g of a powdery yeast extract, containing 15.0 % by weight of 5'-IMP and 12.1 % by weight of 5'-GMP was obtained. Each 5'-nucleotide was determined in the same manner as the one described in Example 3.

A 1 % aqueous solution thereof had a flavor wherein the amino acid and peptide-like taste originating from the yeast was blended with deliciousness similar to those of dried bonito and shiitake mushroom. This flavor was more delicious than those of Examples 3 and 4.

Example 6

The procedure of Example 3 was followed except that no Deamizyme was added. Thus a yeast extract containing 12.5 % by weight of 5'-GMP was obtained. The product had no dry bonito-like flavor but an intense one similar to that of shiitake mushroom.

Comparative Example 1

Candida utilis CS 7529 cells were treated in the same manner as the one described in Example 3 except that the thermal treatment at 80 to 100°C prior to the extraction was omitted.  Thus approximately 65 g of a powdery yeast extract was obtained from approximately 300 g of the cells on a dry basis.

The obtained extract contained 5'-IMP, 5'-GMP, 5'-CMP and 5'-UMP each in an amount less than 0.5 % by weight.  A 1 % aqueous soltuion thereof showed an amino acid-like flavor but little deliciousness.

Comparative Example 2

Candida utilis CS 7529 cells were treated in the same manner as the one described in Example 3 except that the liquid temperature was not adjusted to 50°C at the addition of the 5'-phosphodiesterase but the enzyme was added at room temperature.  The resulting mixture was heated to 65°C and the enxymatic reaction was conducted at this temperature.  Thus approximately 75 g of a powdery yeast extract was obtained from approximately 300 g, on a dry basis, of the cells.  This extract contained 2.5 % by weight of 5'-IMP, 1.7 % by weight of 5'-GMP, 1.3 % by weight of 5'-CMP and 2.5 % by weight of 5'-UMP.  The flavor of a 1 % aqueous solution thereof was inferior in deliciousness to that of Example 3.

Industrial Applicability:

According to the process of the present invention, RNA contained in RNA-rich yeast cells can be converted not into 2'- or 3'-nucleotides but exclusively into 5'-nucleotides by preliminarily heating the cells. In this process, it is unnecessary to use a large amount of alkalis, common salt or surfactants, unlike in the conventional processes. Thus the whole extract thus obtained can be applied as such as a seasoning of an excellent flavor.

A yeast extract obtained from a mutant yeast strain containing 10 % or more, preferably 15 % or more of RNA, has a particularly delicious flavor.

Reference to Microorganism Deposited According to Rule 13-2:

1. Candida utilis CS 7529

a. Name and address of the organization with which the above microorganism has been deposited:

   Name: Fermentation Research Institute, Agency of Industrial Science and Technology.

   Address: 1-3, Higashi 1 chome, Tsukuba-shi, Ibaraki-ken 305, JAPAN.

b. Deposition Date: January 18, 1988.

   This strain has been changed from FERM No. 3340 deposited in Japan.

c. Deposition No.: FERM BP-1656.

2. Candida utilis CSB 6316

a. Name and address of the organization with which the above microorganism has been deposited:

Name: Fermentation Research Institute, Agency of Industrial Science and Technology.

Address: 1-3, Higashi 1 chome, Tsukuba-shi, Ibaraki-ken 305, JAPAN.

b. Deposition Date: January 18, 1988.

c. Deposition No.: FERM BP-1657.

2∂  0299078

<u>T R A N S L A T I O N</u>

INTERNATIONAL FORM

BUDAPEST TREATY ON THE INTERNATIONAL
RECOGNITION OF THE DEPOSIT OF MICRO-
ORGANISMS FOR THE PURPOSES OF PATENT
PROCEDURE

RECEIPT IN THE CASE OF AN ORIGINAL DEPOSIT

issued pursuant to Rule 7.1 by the INTERNA-
TIONAL DEPOSITARY AUTHORITY identified at
the bottom of this page.

Depositor

| | |
|---|---|
| Name | Kohjin Co., Ltd.<br>Administrator: Hajime Muraki |
| Address | 1-1, Shinbashi 1-chome, Minato-ku, Tokyo |

---

I.   Indication of Microorganism

(Depositor's designation for identification)  Deposit received
number

Candida Utilis CSB6316

FERM BP-1657

---

II.   Scientific Properties and Taxonomical Classification

The microorganism indicated in Item I was accompanied
with the following documents:

[X]   Scientific properties
[X]   Taxonomical classification

---

III.   Receipt and Acceptance of Deposit

This international depositary authority accepts deposit on
January 18, 1988 (date of original deposit) of micro-
organism indicated in Item I.

---

IV.   International Depositary Authority

Fermentation Research Institute
Agency of Industrial Science and Technology

Tomoo Suzuki      Dr.  Director General
1-3, Higashi 1 chome, Tsukuba-shi,
Ibaraki-ken
305, Japan

January 18, 1988

TRANSLATION

INTERNATIONAL FORM

BUDAPEST TREATY ON THE INTERNATIONAL
RECOGNITION OF THE DEPOSIT OF MICRO-
ORGANISMS FOR THE PURPOSES OF PATENT
PROCEDURE

RECEIPT IN THE CASE OF AN ORIGINAL DEPOSIT

issued pursuant to Rule 7.1 by the INTERNA-
TIONAL DEPOSITARY AUTHORITY identified at
the bottom of this page.

Depositor

Name       Kohjin Co., Ltd.
           Administrator: Hajime Muraki
Address    1-1, Shinbashi 1-chome, Minato-ku, Tokyo

---

I.    Indication of Microorganism

(Depositor's designation for identification)    Deposit received number

     Candida Utilis Cs 7529                 FERM BP- 1656

---

II.   Scientific Properties and Taxonomical Classification

The microorganism indicated in Item I was accompanied
with the following documents:

     [X]    Scientific properties
     [X]    Taxonomical classification

---

III.   Receipt and Acceptance of Deposit

This international depositary authority accepts deposit on
May 1, 1981      (date of original deposit) of micro-
organism indicated in Item I (transferred custody from
Fermentation Research Institute deposit No. 3340 depo-
sited on December 11, 1975.

---

IV.   International Depositary Authority

        Fermentation Research Institute
      Agency of Industrial Science and Technology

    Tomoo Suzuki      Dr. Director General

    1-3, Higashi 1 chome, Tsukuba-shi,
            Ibaraki-ken
            305, Japan

                   January 18, 1988

## Claims

1. A process for preparing a natural yeast extract rich in 5'-GMP, which comprises heating an aqueous solution containing living yeast cells rich in RNA to 80 to 120°C, extracting the RNA components, and then treating the extract with 5'-phosphodiesterase.

2. A process for preparing a yeast extract as set forth in Claim 1, wherein said yeast extract contains 7 % by weight or more of 5'-GMP.

3. A process for preparing a natural yeast extract rich in 5'-IMP and 5'-GMP, which comprises heating an aqueous solution containing living yeast cells rich in RNA to 80 to 120°C, extracting the RNA and extractable components, and treating the extract with 5'-phosphodiesterase and then with deaminase.

4. A process for preparing a yeast extract as set forth in Claim 3, wherein said yeast extract contains 5'-IMP and 5'-GMP each in an amount of 7 % by weight or more.

5. A process for preparing a yeast extract as set forth in Claim 3, wherein said extraction is carried out at a pH value of 8 to 12 and at a temperature of 40 to 50°C.

6. A process for preparing a yeast extract as set forth in Claim 1 or 3, wherein Candida utilis containing 10 % by weight or more of RNA is employed as said yeast cells.

7. A process for preparing a yeast extract as set forth in Claim 6, wherein Candida utilis CS 7529 (FERM BP-1656) is employed as said yeast cells.

8. A process for preparing a yeast extract as set forth in Claim 6, wherein Candida utilis CSB 6316 (FERM BP-1657) is employed as said yeast cells.

9. A yeast extract containing 7 % by weight or more of 5'-GMP optionally together with 7 % by weight or more of 5'-IMP, which is obtained by treating an extract of yeast cells with enzyme(s) without adding any corresponding component(s) from the outside of the system thereto.

10. Candida utilis CSB 6316 mutant (FERM BP-1657) which is resistant to boric acid and contains a large amount of RNA.

# FIG. 1

DETERMINATION OF 5'-NUCLEOTIDES
BY HIGH PERFORMANCE LIQUID
CHROMATOGRAPHY

0299078

# INTERNATIONAL SEARCH REPORT

International Application No    PCT/JP88/00044

**I. CLASSIFICATION OF SUBJECT MATTER** (if several classification symbols apply, indicate all) 3

According to International Patent Classification (IPC) or to both National Classification and IPC

Int.Cl$^4$    A23L1/28, 1/229

**II. FIELDS SEARCHED**

| Minimum Documentation Searched 4 | |
| --- | --- |
| Classification System | Classification Symbols |
| IPC | A23L1/229, 1/23, 1/28 |

Documentation Searched other than Minimum Documentation
to the Extent that such Documents are Included in the Fields Searched 6

**III. DOCUMENTS CONSIDERED TO BE RELEVANT** 14

| Category * | Citation of Document, 16 with indication, where appropriate, of the relevant passages 17 | Relevant to Claim No. 18 |
| --- | --- | --- |
| A | JP, B1, 43-28831 (Sanyo Pulp Co., Ltd.) 11 December 1968 (11. 12. 68) (Family: none) | 1-5 |
| A | Agric. Biol. Chem., Vol. 38, No. 4 (1974) T. Yamashita et al., [Studies on Substances which increase RNA Content of Yeast Cells] P.724-734 | 1-10 |
| A | JP, A, 52-90684 (Kohjin Co., Ltd.) 30 July 1977 (30. 07. 77) & JP, B2, 81-46824 | 1-10 |

* Special categories of cited documents: 16

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search 2 | Date of Mailing of this International Search Report 2 |
| --- | --- |
| April 8, 1988 (08. 04. 88) | April 25, 1988 (25. 04. 88) |
| International Searching Authority 1 | Signature of Authorized Officer 20 |
| Japanese Patent Office | |